# EUROPEAN PATENT APPLICATION

(11) **EP 4 279 123 A1**
(43) Date of publication of application: **22.11.2023**
(21) Application number: 22174485.7
(22) Date of filing: 20.05.2022
(51) Int. Cl.: A61N 1/365, A61N 1/368, A61N 1/375

(54) **PACEMAKER AND OPERATION METHOD OF SUCH PACEMAKER**

(71) Applicant: BIOTRONIK SE & Co. KG, 12359 Berlin (DE)
(72) Inventor: Whittington, R., Hollis, Portland, 97202 (US); Kraetschmer, Hannes, West Linn, 97068 (US); Swenson, Kurt, Dayton, 97114 (US); Taff, Brian, M., Portland, 97217 (US)
(74) Representative: Biotronik Corporate Services SE

(57) **Abstract**

The invention is generally directed to a cardiac pacemaker (10) which stays synchronized with the heart's natural cycle but also provides a safe patient support in cases where an atrial rhythm is detected by the implant promoting suspicion that an SVT occurred. The pacemaker comprises a processing unit (120), a detector (126) and a pacing signal generator (124), wherein the detector (126) and the pacing signal generator (124) are electrically connected to the processing unit (120), wherein the processing unit (120) is configured to determine a ventricular pacing time value and/or a ventricular pacing rate value and to transmit a corresponding pacing information to the pacing signal generator (124) for providing a pacing signal for a patient's heart (20) based on this information.

The invention is further directed to an operation method of such a pacemaker, a respective computer program product and computer readable data carrier.

## Description

The invention is generally directed to a cardiac pacemaker and an operation method of such pacemaker, a respective computer program product and computer readable data carrier.

A cardiac pacemaker (or artificial pacemaker) is a medical device that generates electrical pulses delivered by electrodes connected to or fixed at the pacemaker to cause the heart muscle chambers (i.e., the atria and/or the ventricles) to contract and therefore pump blood. By doing so this device replaces and/or regulates the function of the electrical conduction system of the heart. One purpose of a pacemaker is to maintain an adequate heart rate (cardiac rate), either because the heart's natural pacemaker is not fast enough, or because there is a block in the heart's electrical conduction system. Additionally or alternatively, the pacemaker may stimulate different positions within the ventricles to improve the synchronization of the ventricles or provide anti-tachycardia functions in order to treat life-threatening arrhythmias. Modern pacemakers are externally programmable and allow a health care practitioner (HCP), such as a clinician, to select the optimal pacing mode(s) for individual patients.

A conventional pacemaker comprises a controlling and generator device comprising a processing unit and a power source external of the patient's heart and electrodes that are implanted within the heart's muscle. The electrodes are connected via leads and a header located at the device to the device. In most cases, the device is implanted subcutaneously in the front of the chest in the region of the left or right shoulder. An implantable intra-cardiac pacemaker (also known as an implantable leadless pacemaker - ILP) is a miniaturized pacemaker which is entirely implanted within a patient's heart ventricle (V) or atrium (A) and serves as a critical development paradigm for the future of cardiac rhythm management. Alternative or additional functions of conventional or intra-cardiac pacemakers comprise providing other electrical or electromagnetic signals to the heart or its surrounding tissue and sensing electrical or electromagnetic signals (e.g., signals from electrical depolarization fields) or other physiological parameters of the heart and/or its surrounding tissue such as the intrinsic (i.e., the heart's natural) atrial contraction or the intrinsic ventricular contraction. Due to the highly restricted device size, an ILP has a reduced battery capacity compared to legacy approaches uniquely challenging design flexibilities for realizing safe and effective bradycardia symptom remediation therapies subject to clinically-critical product service times.

An ILP may be operated in VDD pacing mode (i.e., a pacing mode in which the ventricle is stimulated according to the intrinsic atrial signal and AV conduction monitoring). In the VDD mode, the pacemaker synchronizes ventricular pacing with the intrinsic atrial timing by sensing when atrial contractions (i.e., the intrinsic atrial signals) occur. In an ILP that is implanted in the right ventricle, the atrial contraction information can be detected, but with less reliability and accuracy than in a dual chamber conventional pacemaker where there is a lead in the right atrium as well as the right ventricle.

The pacing functionality of a conventional cardiac pacemaker or an ILP is aimed at staying synchronized as far as possible with the heart's natural activity whenever doing so effectively supports sufficient cardiac output.

The utilization of single chamber ILP requires implants to render some of the same therapy offerings as conventional pacemaker designs. As such, a new generation of ILP incorporating capabilities is desired that effectively "do more" than their legacy-device equivalents by driving output on the basis of both in-chamber and remotely sensed heart activities. As an example, the detection of atrial far field signals needs to be utilized to provide reliable AV synchronous pacing with a single implant in the right ventricle. This needs to be done in a manner that avoids synchronizing to suspected supraventricular tachycardias (SVT) (i.e., abnormally high heart rates originating in the atria or AV node). Presently, ILPs offer a means for sensing of remote atrial heart activity that utilizes an accelerometer in support of an AV synchronous VDD-like therapy. This implementation is not, however, capable of recognizing SVT-like rhythms.

Accordingly, there is the need for a cardiac pacemaker that stays synchronized with the heart's natural cycle but also provides a safe patient support in cases where an atrial rhythm is detected by the implant promoting suspicion that an SVT occurred. Similarly, a corresponding operation method of such pacemaker is needed.

The above problem is solved with a cardiac pacemaker comprising the features of claim 1 and an operation method comprising the features of claim 8 as well as with a computer program product comprising the features of claim 14 and computer readable data carrier comprising the features of claim 15.

In particular, the above problem is solved by a cardiac pacemaker comprising a processing unit, a detector and a pacing signal generator, wherein the detector and the pacing signal generator are electrically connected to the processing unit. The processing unit is further configured to determine a ventricular pacing time value and/or ventricular pacing rate value and to transmit a corresponding information to the pacing signal generator for providing a pacing signal for a patient's heart based on this information. The processing unit further comprises a motion flag reflecting the actual activity of the patient, an actual status of which is either true during high activity or false during lower activity and/or zero activity of the patient. The detector is configured to capture time-dependent signals of the cardiac activity containing intrinsic atrial events or evidence of such events from conducted intrinsic ventricular events of the patient's heart. In a first atrial tracking state, the processing unit is configured to continuously determine an estimated cardiac rate based on at least one actual intrinsic atrial event or evidence of such an event from a conducted intrinsic ventricular event detected from the received cardiac activity signals and to compare the estimated cardiac rate with a pre-defined validity check rate, wherein if the estimated cardiac rate is greater than or equal to the validity check rate the processing unit is configured to assess the actual status of the motion flag.
- If the estimated cardiac rate is greater than or equal to the validity check rate and the actual status of the motion flag is true or if the estimated cardiac rate is lower than the validity check rate, the processing unit is configured to stay in the first atrial tracking state in which the ventricular pacing time value and/or the ventricular pacing rate value is determined based on the estimated cardiac rate.
- If the estimated cardiac rate is greater than or equal to the validity check rate and the actual status of the motion flag is false, the processing unit is configured to transition into a supraventricular tachycardia state (SVT state) in which the processing unit is configured to ramp the ventricular pacing time value up and/or the ventricular pacing rate value down until a pre-defined first pacing time threshold value and a pre-defined first pacing rate threshold value, respectively, is reached, and to suspend detecting intrinsic atrial events of the patient's heart from the received cardiac activity signals. For example, approaching the pre-defined first pacing rate threshold value (i.e., a resting or idle rate) the ventricular pacing rate is ramped down using a decay rate with nominal fixed decay (e.g., rate degraded by 0.5 bpm per therapy output cycle) until the pre-defined first pacing rate threshold value is reached.

The pacemaker may be a conventional cardiac pacemaker or a defibrillator with pacing functionality that uses leads or an ILP having the general structure as indicated above and below.

With regard to the invention, the processing unit is generally regarded as a functional unit of the pacemaker, that interprets and executes instructions comprising an instruction control unit and an arithmetic and logic unit. The processing unit may comprise a microprocessor, a controller, a digital signal processor (DSP), an application specific integrated circuit (ASIC), a field-programmable gate array (FPGA), discrete logic circuitry or any combination thereof. Alternatively, or additionally, the processing unit may be realized using integrated dedicated hardware logic circuits, in particular, in the case of an ILP due to the small size and extreme power limitation.

The processing unit processes signal data received from the detector, for example a motion signal and an electrical signal which are detected over time. Thus, the detector may provide more than one signal, and/or there may be more than one detector or one single detector. In particular, the detector may be configured to capture time-dependent signals of the cardiac activity (i.e., signaling that may involve electrical/electromagnetic, auditory, or other inputs capable of reporting myocardial polarization states or otherwise). From these signals the detector may derive electrical signals such as electrical signals associated with the contraction of one of the atria (in the following intrinsic atrial event) and electrical signals associated with the contraction of the ventricles (in the following intrinsic ventricular event). In the case of an ILP, the intrinsic atrial event may be a far field signal. The detector may further be configured to differentiate between intrinsic atrial events and intrinsic ventricular events.

The ILP or the conventional pacemaker may be operated in the first state according to the VDD pacing mode (i.e., a generally known pacing mode in which the ventricle is stimulated according to atrial activity and AV conduction monitoring). In the VDD mode, the pacemaker synchronizes ventricular pacing with the intrinsic atrial event (atrial contraction). It may further use the intrinsic ventricular event (ventricular contraction) and/or the ventricular pacing signal to determine the ventricular pacing time value and/or the ventricular pacing rate value. In an ILP that is implanted in the right ventricle, the atrial contraction information is detected as a far-field signal as indicated above. The ventricular pacing time value and/or the ventricular pacing rate value is further determined based on an estimated cardiac rate which is determined from the cardiac events as indicated below in the first state. Alternatively, the processing unit may be operated in a further state in the VVI or VVI-R mode.

The processing unit may further comprise a counter and a clock (VDD timer). The counter may be used to count clock signals of the clock. The counter may be started at each sensed atrial or ventricular contraction and count the number of clock signals until the next atrial or ventricular contraction occurs or ventricular pacing is provided by the pacing signal generator. In the first state, the processing unit may determine from the intrinsic atrial events and, if applicable, the intrinsic ventricular events the estimated cardiac rate of the patient. The estimated cardiac rate may be used to determine the ventricular pacing time value and/or the ventricular pacing rate value and to provide a pacing information being a pacing control signal (e.g., the ventricular pacing control signal) reflecting the ventricular pacing time value and/or the ventricular pacing rate value, wherein the pacing information may then be transmitted to the pacing signal generator. Further, the pacing information may contain/reflect a synchronization with the intrinsic atrial events determined by the detector. The pacing information may be, for example, the information about the administration time as well as the amplitude/intensity, duration, form information of the pacing signal to be administered.

Based on the pacing information received from the processing unit the pacing signal generator produces the electrical pacing signal(s) in order to transfer it to the electrodes which apply the signal(s) to the heart's tissue adjacent to the electrode. The pacing signals are pulses that begin at a desired time point and have a desired intensity and duration. Further, the pulse form may be varied. Information necessary to produce the correct pacing signals are provided by the processing unit based, at least in part, on the ventricular pacing time value and/or the ventricular pacing rate value. In one embodiment, the pacing signal is not determined by the pacing signal generator or not transferred to the electrodes if an intrinsic ventricular signal is detected within a predefined time period after the detected intrinsic atrial event (i.e., an inhibited condition).

The pacemaker may comprise a data memory which may include any volatile, non-volatile, magnetic, or electrical media, such as a random access memory (RAM), read-only memory (ROM), non-volatile RAM (NVRAM), programmable ROM (e.g. EEPROM), flash memory, or any other memory device. The data memory saves the above and below mentioned thresholds, values, flags, parameters and conditions. They are required by the processing unit during processing the above and below explained steps.

The at least one detector further comprises, in addition to the above functions, an accelerometer, a vibration sensor, an acoustic sensor (including ultrasound) and/or any other mechanical, electric and/or magnetic sensor that is capable of detecting the actual patient activity as a function of time (i.e., a patient body motion sensor). This capability serves to offer dynamic insight on matters related to whether the patient is moving or stationary, for example monitoring for changes in body position/posture, sleeps states, and/or specific mobility/exercise conditions. The detector collects the activity signals of the patient and transforms them into electrical signals. Generally, the detector may digitize all or some detected or measured analog signals, filter them and/or smooth them to reduce signal noise and/or cull specific metrics. Some pre-processing steps may be provided by the detector, as well. The time dependent motion signal produced by the detector is preferably transmitted to the processing unit directly.

The pacemaker's units and components may be contained within a hermetically sealed housing.

In one embodiment, the pacemaker comprises electrodes for application of an electrical pacing signal provided by the pacing signal generator. The electrodes are electrically connected to the pacing signal generator via a header of the pacemaker. In one embodiment (i.e., in cases where the pacemaker is a conventional pacemaker) the electrode may comprise a lead which may be detachably connected to the respective connector at the header. If the cardiac pacemaker is an ILP one electrode may be located at a distal end of the ILP, close to a fixation member by which the ILP is fixed in the tissue of the patient's heart, for example within the inner tissue of a ventricle. A second electrode may be located at the proximal end of the ILP or a part of the ILP housing that may, for example, serve as counter electrode. Further, the electrodes may be adapted to detect the intrinsic ventricular signals and/or the intrinsic atrial signals over time by picking up electrical potentials. The electrodes may thereby be part of the detector of the pacemaker.

The cardiac rate of the patient is determined continuously or periodically via interval assessments based, at least in part, on the intrinsic atrial events detected by the detector. Additionally, intrinsic ventricular events detected by the detector may be used to determine, again, at least in part, this estimated cardiac rate. If, in one cycle no atrial signal is detected, the most recent estimated cardiac rate of a previous one or more sufficiently recent cardiac cycles may be used as the (actual) estimated cardiac rate. Additionally or alternatively, the estimated cardiac rate may be determined as a mean of a pre-defined number of previous cardiac cycles and, if applicable, of the actual cardiac cycle.

In the first state, the estimated cardiac rate determined by the processing unit based on the intrinsic atrial signal is periodically or continuously compared with a pre-defined validity check rate (examples for validity check rates - see below). This check is performed to determine whether an SVT may or may not be in effect. If the estimated cardiac rate is less than the validity check rate, the processing unit further operates in the first state as no SVT seems to be present at this moment. However, if the estimated cardiac rate is greater than or equal to the validity check rate, the processing unit is configured to assess the status of a motion flag which is either true, during high activity of the patient, (e.g. if the patient's detected body movements are equal to or exceed a pre-defined motion level), or false, subject to low/no activity conditions (i.e., the patient's movements reside below a pre-defined motion level) wherein the processing unit is configured to determine the status of the motion flag based on the recently detected motion signal of the detector reflecting the actual activity of the patient. If the estimated cardiac rate is greater than or equal to the validity check rate grounds exist for determining whether or not an SVT is in effect. Accordingly, it is checked whether an activity of the patient is the reason for the increased estimate cardiac rate. Dependent on this additional check the processing unit continues to control pacing using the first state or switches to the SVT state described above and below.

The motion flag status is set subject to conditions in the first state where the motion sensor of the detector (e.g. the accelerometer) is turned to an ON state and used for determining the motion signal. Such conditions occur, for example if the processing unit is operating in a sensor-driven mode/state (i.e., key "mode switched" sub-states of leadless-specific VDD mode embodiments or VVI-R) or in cases, as mentioned above, where atrial tracking conditions are being benchmarked, via triggers or periodic checks, to determine whether or not tracked rates are associated with an affiliate degree of patient motion (i.e., subject to interactions with the validity check rate). The processing unit continuously or periodically determines the actual activity of the patient based on the detected actual signal of the motion sensor and uses such gathered data to set the status of the motion flag.

In one embodiment, in either the mode/state or check conditions, if the sensor rate found by the accelerometer is equal to or exceeds a motion threshold that represents "sufficient" patient body motion (i.e. a predefined patient activity level), the patient is deemed to be in motion and the motion flag is set to a true status. Otherwise, the motion flag is set to a false status. The output of the accelerometer used for detecting motion will span a range of values wholly dependent upon the specific part used and the range of proof-mass motion that can occur in such parts. This full range of signaling is typically subsequently digitized using an analog-to-digital converter (ADC) that, in turn, establishes numerical codes for the full range of possible proof mass signal conditions stemming from the motion of the proof mass. By sampling the digitized values emergent from the ADC, a time series of acceleration values can be acquired by the sensor/processing unit to quantify the motion of the patient (weening out cardiac wall motion through appropriate band-pass signaling). In one embodiment, the processing unit or the detector periodically samples the signaling from this ADC (e.g., one sample per second) for a specified duration (e.g., for four (4) samples) and quantifies the ADC values at each of such sample points. Then a criterion applied by the system may determine if the content within the collected samples was indicative of "sufficient" motion. Such may be realized by a so-called "X out of Y" condition where, for example 3(X) out of 4(Y) collected motion samples would present ADC values greater than ones empirically determined though clinical research efforts to prove indicative of patient body motion outside of an "at rest" condition. The specific number associated with such a point would be highly dependent upon the sensor chosen, it's sensitivity, and the device and clinical context in which it resides. If, on the other hand the sensor rate associated with the accelerometer is below the motion threshold representing "sufficient" patient body motion (e.g., if none, 1, or 2(X) out of 4(Y) ADC values exceeded a threshold greater than one indicative of a patient at rest), the patient is deemed to be inactive and the status of the flag is set to false (e.g. set into the OFF state). The motion threshold may, for example, typically prove representative of a "slow to moderate walking condition" (i.e., an ADC value threshold that may represent something on the order of 20% of the full range of possible ADC values, for example - again, part and system design dependent). In one embodiment, the value for such a condition may, for example, represent something that is not patient-specific and may represent a constant across all devices of a specific build deployed in the field. Setting of this motion threshold would, in one embodiment, be configurable, though remain un-changed once the proper value was established.

If, in the first state, the estimated cardiac rate is greater than or equal to the validity check rate and the motion flag has (currently) the true status, no SVT is suspected. Accordingly, in this case, the processing unit continuously operates in the first state, wherein the first state is now referred to as the "checked" first state. After a pre-defined time period the check is regarded outdated and the comparison with the validity check rate is repeated/re-enabled.

If, however, the estimated cardiac rate is greater than or equal to the validity check rate and the (actual) status of the motion flag is false, it is assumed that the patient's heart has developed an SVT. Accordingly, the processing unit is configured to transition into an SVT state (the SVT state may also be referred to as fading state, whereby the fading to non-tracking may be just one part of the SVT state). In this state, ventricular pacing should not follow the intrinsic atrial signal. Rather, according to the invention, the processing unit is configured to determine the ventricular pacing time value and/or the ventricular pacing rate value independently from the intrinsic atrial signal. For example, the ventricular pacing rate value (the administered therapy rate) is ramped down (e.g., decaying at a nominal fixed rate of 0.5 bpm per therapy output/cardiac cycle, analogously the ventricular pacing time/period value may be ramped up), wherein the initial value may be the most recently determined estimated cardiac rate (or a ventricular pacing time value corresponding to the estimated cardiac rate). The determination of the ventricular pacing rate value/ventricular pacing time value based upon the intrinsic atrial event is suspended until a pre-defined first pacing rate threshold value (also referred to as resting rate, for example, a rate configurable by the HCP and one that would likely represent 60 bpm or some similar nominal rate well aligned with conditions where a patient would be minimally active) is reached and furthermore only after a pre-defined timeout (i.e., a pre-defined first hold-off time period that allows for the termination of the SVT) has occurred. The ventricular pacing rate value/ventricular pacing time value used during the SVT state is determined (at least for portions of the SVT state) based on the ramped estimated cardiac rate (or analogous ramped time value). The rate may be determined furthermore (for different portions of the SVT state) by different sources depending on whether one is solely ramping to a pre-defined first pacing rate threshold value (determined by the fixed decay condition in the fading sub-state) or whether one has decided to "use motion" inputs from the in-device accelerometer to drive therapy output. If one decides to not "use motion", once the pre-defined first pacing rate threshold value is reached, this rate value sets the ventricular pacing rate value (analogously, the pre-defined first pacing time threshold sets the ventricular pacing time value). If one decides to "use motion", once the pre-defined first pacing rate threshold value (or the pre-defined first pacing time threshold value) is reached, the motion-dependent sensor rate value (or a motion-dependent sensor time value) drives the ventricular pacing rate value (or, analogously, the ventricular pacing time value) as indicated below.

In one embodiment, in the SVT state (referring to an SVT suspicion condition) additionally the processing unit is configured to instruct the detector to suspend the detection of the intrinsic atrial event. Accordingly, the detector may be configured to suspend monitoring of the atrial cardiac activity. As the intrinsic atrial event is not needed for the determination of the ventricular pacing time value or the ventricular pacing rate value, this event need not be determined, and, in turn, the system can best conserve its limited power/energy resources in administering therapy. In this and all further embodiments, the SVT state may comprise or be the SVT suspicion condition. Said alternatively, the SVT state or SVT suspicion condition may comprise a multitude of sub-states that, in coordination, serve to manage therapy output when tracking of the atrium has been determined as suboptimal.

In one embodiment, in the SVT state is configured to suspend the capture of the time-dependent signals of the atrial cardiac activity.

In one embodiment, in the SVT state, after the ventricular pacing time value and/or the ventricular pacing rate value has reached the first pacing time threshold value and the first pacing rate threshold value (or resting rate RR), respectively, the processing unit is configured to transition into a non-tracking state, wherein in the non-tracking state the processing unit is configured to continue suspending the detection of intrinsic atrial events of the patient's heart from the received cardiac activity signals and/or to continue suspending the capture of the time-dependent signals of the atrial cardiac activity by the detector. The non-tracking states are, for example, the non-tracking state and the non-tracking with motion state. Accordingly, in this embodiment the processing unit is configured to fade to either the non-tracking state or the non-tracking with motion state where atrial sense inputs are turned off or left unmonitored (i.e., are non-tracked). In one embodiment, these inputs/detections were already turned off when the SVT state (e.g. the initial substate of an SVT suspicion condition, the fade-to-non-tracking state) was entered. It is predefined or user-selectable (user comprises the patient and the HCP) whether the non-tracking state or non-tracking with motion state is used, wherein in the non-tracking states the processing unit is configured to continue suspending the determination of the estimated cardiac rate from the intrinsic atrial event or instructing the detector to suspend the detection of the intrinsic atrial event so that a determination of the estimated cardiac rate from the intrinsic atrial event is not provided thereby not consuming additional energy. The user-selectivity of the state may be provided by using a non-tracking-mode parameter which may have at least two pre-defined different values.

In one embodiment, the processing unit is configured to stay in the SVT state (including, for example, the fading state, the non-tracking state and/or the non-tracking with motion state) at least for a pre-defined first hold-off time period. The pre-defined first hold-off time period is configurable by the user (potentially even by the patient), and lasts, for example, for durations of at least 5-15 minutes whether measured as pure time durations or though cardiac cycle counts as initiated once the system first enters the SVT state or reaches the pre-defined first pacing time threshold value (or the pre-defined first pacing rate threshold value) after entering the SVT. This predefined first hold-off time period effectively amounts to the duration the system may reside within either the non-tracking or non-tracking with motion states before attempting to transition to other downstream system states. In the case where the HCP had configured the system to not "use motion", expiration of the first hold-off time period may cause the system to enter a FindSync state (see below). In the case where the HCP had configured the system to "use motion" expiration of the first hold-off time period may cause the system to enter a FindSync once the therapy rate matches that prescribed by the user for FindSync state operation. In one embodiment, in the non-tracking state the processing unit is configured to operate in the VVI mode and in the non-tracking with motion state the processing unit is configured to operate in the VVI-R mode. Accordingly, in one embodiment, in the non-tracking state the processing unit is configured such that the ventricular pacing time value and/or the ventricular pacing rate value is determined using a VVI behavior, if the non-tracking-mode parameter has a pre-defined first value, or using a VVI-R behavior, if the non-tracking-mode parameter has a pre-defined second value different from the first value. The VVI behavior and VVI-R behavior are basically known to the skilled person.

In one embodiment, the processing unit is configured such that the ventricular pacing time value and/or the ventricular pacing rate value is determined using the VVI-R behavior based on the recently detected and received motion signal provided by the detector and/or based on the actual status of the motion flag and such that the ventricular pacing time value and/or the ventricular pacing rate value is determined using the VVI behavior based on a predefined second pacing time threshold value and a pre-defined second pacing rate threshold value, respectively. For example, the ventricular pacing time value is equal to the pre-defined second pacing time threshold value and/or the ventricular pacing rate value is equal to the pre-defined second pacing rate threshold value in the non-tracking state (without motion). In the non-tracking with motion state the ventricular pacing time value may vary between a maximum and a minimum pacing time value and/or the ventricular pacing rate value may vary between a maximum and a minimum pacing rate value, in each case dependent on the recently detected and received motion signal and/or based on the actual status of the motion flag.

However, in one embodiment, if the motion flag changes during one of the non-tracking states, this does not initiate tracking nor termination of the non-tracking state. In one embodiment the first pacing time threshold value may be equal to the second pacing time threshold value or different. Accordingly, the first pacing rate threshold value may be equal to the second pacing rate threshold value or different.

In one embodiment, the selected one of the non-tracking states remains active for durations of, for instance, no less than 15 min following the last/preceding suspected SVT condition was realized. Preferably, the duration of the pre-determined first hold-off time period provides an opportunity for the SVT to terminate and also avoids excessively frequent rate oscillations stemming from any subsequent tracking responses of a present SVT which could rapidly drive the ventricular pacing periods downward or the ventricular pacing rate value upward to engage with the validity check rate. At the same time, it may be important that the validity check rate be low enough, for example between 80 bpm (beats per minute) and 90 bpm, to avoid inducing symptoms that could emerge from tracking SVTs that had not terminated. After the first hold-off time period expires (and the rate matches the resting rate used during FindSync when in non-tracking states that utilize motion), the processing unit is nominally configured to enter the FindSync state. There, support for detection of intrinsic atrial events is resumed.

Alternatively or additionally, in one embodiment, in the non-tracking state after the ventricular pacing time value and/or the ventricular pacing rate value has not exceeded or undercut the first or second pacing time threshold value and the first or second pacing rate threshold value, respectively, for a pre-defined second hold-off time period, the processing unit is configured to transition the pacemaker into a state that attempts to reestablish atrial synchrony, wherein the detection of intrinsic atrial events of the patient's heart from the received cardiac activity signals and/or the capture of the time-dependent signals of the atrial cardiac activity is resumed, and/or at least one AV delay is initiated by at least one detected atrial event and/or detected atrial events initiate AV delays in the pacemaker.

Further, in one embodiment, the detector is further configured to capture time-dependent signals of the cardiac activity containing intrinsic ventricular events, wherein the processing unit is configured such that it synchronizes the pacing signals provided by the pacing signal generator by means of AV delays in response to detected intrinsic atrial events in a transition state which is adopted after leaving the non-tracking state and prior entering the first atrial tracking state, wherein in the transition state the processing unit is configured to resume the detection of intrinsic atrial events of the patient's heart from the received cardiac activity signals and/or the capture of the time-dependent signals of the atrial cardiac activity.

In one embodiment the continuation in the tracking state may be provided after synchronizing the intrinsic heart's activity and the pacing using the FindSync state described below.

In one embodiment, the processing unit is configured such that in the non-tracking state or in the non-tracking with motion state the hold-off time period is prolonged by a pre-defined or user-selectable additional hold-off time period or until a predefined or user-selectable time point, for example Midnight, if a number of consecutive or total transitions into SVT mode within a span of time (e.g., within the day) is greater than a predefined or user-selectable threshold value.

Alternatively or additionally, the processing unit is configured such that in the non-tracking state or in the non-tracking with motion state the first or second hold-off time period is prolonged by a pre-defined or user-selectable additional hold-off time period or until a predefined or user-selectable time point, if a user-selectable maximum daily allowed number of permissible SVT suspicion conditions is exceeded.

In one embodiment, consecutive transitions into SVT state are assumed if the transition from the first state to the SVT state (as indicated above) occur again within a predefined return time period (e.g. 5 minutes), after the ability to detect synchronous atrial intrinsic events resumes or, alternatively as referenced against the timepoints where the mode support exits non-tracking or non-tracking with motion states. The predefined or user-selectable number of consecutive transitions initiating additional hold-off time periods or an extension of the baseline hold-off time period to a predefined or user-selectable time point may be, for example 2, 5, 10 or 20. In another embodiment the prolongation of the hold-off time period is never used.

In one embodiment, to provide the HCP with a sense of how much SVT burden is present, the overall number of transitions into SVT state (from the first state) per day may be stored in the data memory. This number in a day may be qualified in cases where they reached any upper allowable consecutive count limit value. Any statistics collected by the pacemaker related to the number of transitions into the SVT over one day or several days may be made available at follow-up or through home monitoring service center (HMSC) remote monitoring avenues - the latter demanding a specific patient device for engaging with the pacer and collecting data for relay to a web-accessible database. Accordingly, the pacemaker may comprise a communication unit configured to communicate the collected data (e.g., the number of transitions into SVT state, to the patient device). While the details of such HMSC support are not specific to the present invention, the patient device used to collect data from a deep leadless pacer would be expected to utilized electrically-conducted galvanic, impedance, magnetically inductive, and/or acoustic means for communicating with the implant and relay any collected data to the web-accessible database using either web portals or cellular network technologies.

In one embodiment, after expiration of the first or second hold-off time period in the non-tracking or in the non-tracking with motion state, the processing unit is configured to either directly or indirectly (i.e., following the passage through interim states such as the "Sensor" state) transition into a FindSync state, wherein the processing unit is configured such that it synchronizes the pacing signals provided by the pacing signal generator with detected intrinsic ventricular events and/or intrinsic atrial events in this transition state which is adopted after leaving the non-tracking state and prior to entering a bona fide atrial tracking state, wherein in the FindSync state the processing unit is configured to resume the detection of intrinsic atrial events of the patient's heart from the received cardiac activity signals and/or the capture of the time-dependent signals of the atrial cardiac activity. For this, the processing unit is further configured to capture time-dependent signals of the cardiac activity containing intrinsic ventricular events. Accordingly, the processing unit is configured to return into the first (i.e., atrial tracking) state if the intrinsic atrial event can viably support reliable atrial rate tracking through the entry into the first state. As explained above, the non-tracking state or non-tracking with motion state may be prolonged if the transition from the first state to the SVT state occurs consecutively.

In one embodiment, in the FindSync state the processing unit attempts to track atrial detection. A delay in entering the FindSync state may occur in cases where the holdoff duration times out while the implant is in a non-tracking with motion state and the patient is active.

In one embodiment, in the FindSync state a new timer initiates or is/will be initiated. If this timer expires and the implant has a.) not resynched (remaining in either FindSync or a sensor state - see below), b.) resynched and remained below the validity check rate (in an unchecked atrial tracking state - i.e. the first state), or c.) resynched and passed the check with the validity check rate (in a checked atrial tracking state - the patient seems to be active - i.e., the first state with an active patient) the suspected SVT is deemed to have been terminated. In one embodiment, in the FindSync state, if the device resynchronizes and can track atrial activity, then the processing unit resumes normal tracking behavior (i.e. transitions into the first, atrial tracking state). Otherwise, it remains in FindSync (or in cases where motion support has been enabled and the sensor rate dominates the processing unit periodically checks for the prevalence of motion conditions and transitions to sensor driven behaviors). If the processing unit tracks atrial activity, but the estimated cardiac rate determined based on this activity again climbs past the validity check rate, the same procedure is performed, but the first or second hold-off time period may be different which means that the same steps are performed again but the hold-off time period may be prolonged or extended as indicated above.

In one embodiment, if the atrial intrinsic event is tracked at high cardiac rates and the motion flag is on (i.e., activity of the patient is detected), the estimated cardiac rate may reach an upper tracking rate and stay at this rate exceeding a predefined number of cardiac cycles or time. The processing unit may then enter another non-tracking state, which effectively mimics a VVI/VVI-R mode of operation. Depending on if rate response is enabled or not, the processing unit may transition towards the motion driven rate (VVI-R) or fade down to the resting rate and may transition towards the FindSync state.

In above and below explanation a pre-defined (threshold) value is a value that is preprogrammed in the pacemaker (stored in the data memory) and a user-selectable value is a value that may be changed/adapted to the patient's needs by the HCP or the patient, for example by using a programmer.

The above problem is further solved by an operation method of a cardiac pacemaker, wherein the pacemaker comprises a processing unit, a detector and a pacing signal generator, wherein the detector and the pacing signal generator are electrically connected to the processing unit, wherein the processing unit determines a ventricular pacing time value and/or a ventricular pacing rate value and transmits a corresponding pacing information to the pacing signal generator for providing a pacing signal for a patient's heart based on this information and comprises a motion flag reflecting the actual activity of the patient, an actual status of which is either true during high activity or false during lower activity and/or zero activity of the patient, wherein time-dependent signals of the cardiac activity containing intrinsic atrial events or evidence of such events from conducted intrinsic ventricular events of the patient's heart are captured by the detector,
wherein in a first atrial tracking state continuously an estimated cardiac rate is determined based on at least one actual intrinsic atrial event or evidence of such an event from at least one conducted intrinsic ventricular events detected from the received cardiac activity signals and the estimated cardiac rate is compared with a pre-defined validity check rate, wherein if the estimated cardiac rate is greater than or equal to the validity check rate the actual status of the motion flag is assessed,
- wherein, if the estimated cardiac rate is greater than or equal to the validity check rate and the actual status of the motion flag is true or if the estimated cardiac rate is lower than the validity check rate, the method continues in the first atrial tracking state in which the ventricular pacing time value and/or the ventricular pacing rate value is determined based on the estimated cardiac rate,
- wherein, if the estimated cardiac rate is greater than or equal to the validity check rate and the actual status of the motion flag is false, the method transitions into an SVT state in which the ventricular pacing time value is ramped up and/or the ventricular pacing rate value is ramped down until a pre-defined first pacing time threshold value and a predefined first pacing rate threshold value, respectively, is reached, and the detection of intrinsic atrial events of the patient's heart from the received cardiac activity signals is suspended.

In one embodiment of the operation method, in the SVT state suspends the capture of the time-dependent signals of the atrial cardiac activity.

In one embodiment of the operation method, in the SVT state (additionally the detector) suspends the detection of the intrinsic atrial event.

In one embodiment of the operation method, in the SVT state after the ventricular pacing time value and/or the ventricular pacing rate value has reached the first pacing time threshold value and the first pacing rate threshold value, respectively, the processing unit transitions into a non-tracking state, wherein in the non-tracking state the processing unit continues suspending the detection of intrinsic atrial events of the patient's heart from the received cardiac activity signals and/or continues suspending the capture of the time-dependent signals of the atrial cardiac activity by the detector.

In one embodiment of the operation method, in the non-tracking state the ventricular pacing time value and/or the ventricular pacing rate value is determined using a VVI behavior, if a non-tracking-mode parameter has a pre-defined first value, or using a VVI-R behavior, if the non-tracking-mode parameter has a pre-defined second value different from the first value.

In one embodiment of the operation method, the ventricular pacing time value and/or the ventricular pacing rate value is determined using the VVI-R behavior based on the recently detected and received motion signal provided by the detector and/or based on the actual status of the motion flag and/or the ventricular pacing time value and/or the ventricular pacing rate value is determined using the VVI behavior based on a pre-defined second pacing time threshold value and a pre-defined second pacing rate threshold value, respectively.

In one embodiment, this second pacing time threshold value may equal the pre-defined first pacing rate threshold value.

In one embodiment of the operation method, in the non-tracking state after the ventricular pacing time value and/or the ventricular pacing rate value has not exceeded or undercut the first or second pacing time threshold value and the first or second pacing rate threshold value, respectively, for a pre-defined hold-off time period, the processing unit causes the pacemaker to transition to a state that attempts to reestablish atrial synchrony, wherein the detection of intrinsic atrial events of the patient's heart from the received cardiac activity signals and/or the capture of the time-dependent signals of the atrial cardiac activity is resumed and/or detected atrial events initiate AV delays in the pacemaker.

In one embodiment of the operation method, the processing unit synchronizes the pacing signals provided by the pacing signal generator with detected intrinsic ventricular events and/or intrinsic atrial events in a transition state or FindSync state which is adopted after leaving the non-tracking state (or after leaving the non-tracking state with motion and proceeding through interim states between it and FindSync) and prior to entering the first state (i.e., a bona fide atrial tracking state), wherein in the FindSync state the processing unit initiates resumption of the detection of intrinsic atrial events of the patient's heart from the received cardiac activity signals and/or of the capture of the time-dependent signals of the atrial cardiac activity.

The above embodiments of the operation method have the same advantages as the above pacemaker. Embodiments of the pacemaker indicated above may be realized in the operation method analogously. It is referred to the above explanation of the pacemaker in this regard.

The above method is, for example, realized as a computer program which comprises instructions which, when executed, cause the processing unit (processor) to perform the steps of the above method (to be executed by the medical device, in particular at its processor) which is a combination of above and below specified computer instructions and data definitions that enable computer hardware to perform computational or control functions or which is a syntactic unit that conforms to the rules of a particular programming language and that is composed of declarations and statements or instructions needed for an above and below specified function, task, or problem solution.

Furthermore, a computer program product is disclosed comprising instructions which, when executed by the processing unit, cause the processing unit to perform the steps of the above defined method. Accordingly, a computer readable data carrier storing such computer program product is disclosed.

The above pacemaker, method, computer program and computer program product employ an algorithmic means to adapt therapy output that biases CRM synchronous pacing toward non-arrhythmic conditions and thereby ultimately offers safer patient support. They further maximize patient comfort in VDD-like mode operation and provide enhanced clinical controls for improving patient/pacemaker interactions based on underlying etiology considerations - through configuration support for the level of "hold-off time period" applied for avoiding the repeated detection of persistent SVT suspicion conditions. Additionally, they embed a predictable/controllable response to SVTs that is readily explainable to HCPs/patients. Affiliate statistics may provide insights on the frequency, duration, and counts of suspected SVTs to support improved patient care though subsequent pacemaker/medication adjustments. Furthermore, if paired with targeted HMSC surveys of pacemaker/patient interactions, the above invention offers a means for assessing SVT suspicions between scheduled clinical follow-ups.

The present invention will now be described in further detail with reference to the accompanying schematic drawing, wherein
- Fig. 1: shows a first embodiment of the pacemaker within a cross section of a patient's heart,
- Fig. 2: depicts a functional block diagram of the pacemaker shown in Fig. 1, and
- Fig. 3: shows a flow chart of an embodiment of the operation method of the pacemaker of Fig. 1.

In the following, the invention is described with regard to an ILP. It may analogously be realized in a conventional pacemaker or defibrillator which has a pacing function, as well.

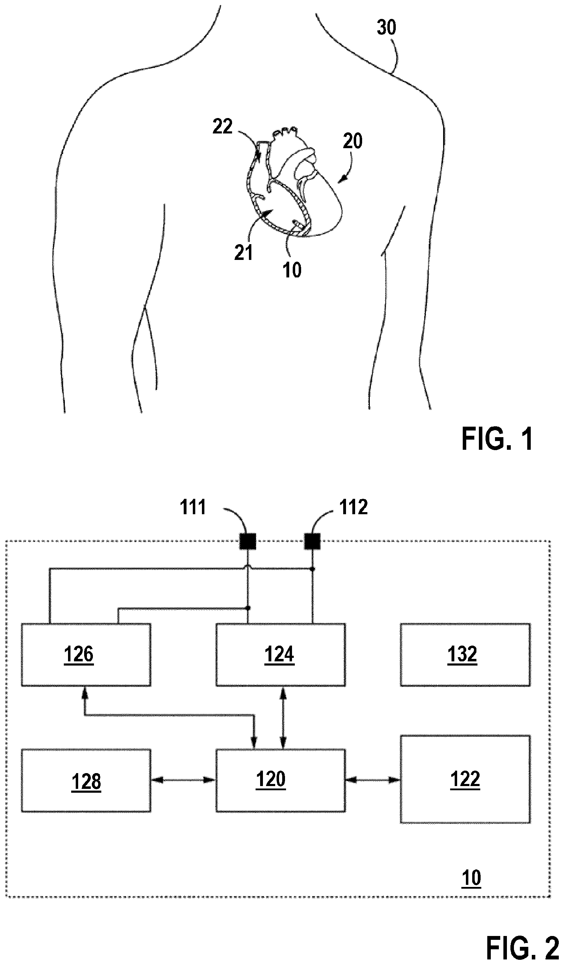

Fig. 1 shows an example leadless ventricular pacemaker (ILP) 10 implanted within the heart 20 of a patient 30. ILP 10 may be configured to be implanted within the right ventricle 21 of the heart 20 and pace this ventricle, sense intrinsic ventricular depolarizations and depolarizations of the atria (e.g. the right atrium 22) and inhibit ventricular pacing in response to detected ventricular depolarization. A programmer (not shown) may be used to program ILP 10 and retrieve data from ILP 10. The ILP 10 is one example of a cardiac pacemaker 10. Other embodiments of the cardiac pacemaker 10 are possible.

Fig. 2 shows a functional block diagram of the ILP 10 configured for implantation within ventricle 21 (Fig. 1). The ILP 10 comprises a processing unit 120 with a clock, at least one counter for the clock signals and a data memory 122, a pacing signal generator 124, a detector 126, a communication unit 128, and a power source 132. The power source 132 may be electrically connected to one or more of the other components/units 120, 122, 124, 126, 128 (not shown in Fig. 2) and may include a battery (e.g., a rechargeable or nonrechargeable battery). The power source 132 provides electrical energy to all units and components of the ILP 10 (not explicitly shown in the figure to keep the figure simple), in particular to all units mentioned above and is therefore electrically connected to these units and components. Units included in ILP 10 represent their respective functionality. Similar or identical units and functionality may also be included in the ILP 10. Units of the pacemaker of present disclosure may include any discrete and/or integrated electronic circuit components that implement analog and/or digital circuits capable of producing the functions attributed to the units herein. For example, the units may include analog circuits, amplification circuits, filtering circuits, and/or other signal conditioning circuits. The units may also include digital circuits (e.g., combinational or sequential logic circuits), memory devices, etc. The units may further be realized using integrated dedicated hardware logic circuits. The data memory 122 may include any volatile, non-volatile, magnetic, or electrical media mentioned above. Furthermore, the processing unit 120 may include instructions that, when executed by one or more processing circuits, cause the units to perform various functions attributed to these units herein. The functions attributed to the units herein may be embodied as one or more processors, hardware, firmware, software, or any combination thereof. Depiction of different features as units is intended to highlight different functional aspects, and does not necessarily imply that such units must be realized by separate hardware or software components. Rather, functionality associated with one or more units may be performed by separate hardware or software components, or integrated within common or separate hardware or software components. Data memory 122 may store computer-readable instructions that, when executed by processing unit 120, cause processing unit 120 to perform the various functions attributed to processing unit 120 herein. Further, data memory 122 may store parameters for these functions (e.g. pacing signal parameters, values, conditions and thresholds described above and below). The pacing instructions and pacing signal parameters, conditions and thresholds may be updated by the programmer using the communication unit 128. The communication unit 128 may comprise an antenna, coil, patient anatomical interface, and/or a transceiver.

The processing unit 120 may communicate with pacing signal generator 124 and detector 126 thereby transmitting signals. Pacing signal generator 124 and detector 126 are electrically coupled to electrodes 111, 112 of the ILP 10. Detector 126 is configured to monitor signals from electrodes 111, 112 to monitor electrical activity of heart 20. Further, the detector 126 may include a motion sensor, for example an accelerometer or any other motion sensor described above. However, an accelerometer-based motion sensor does not necessarily require any connection with the electrodes 111, 112. The motion sensor collects a time-dependent motion signal as described above and transmits this signal to the processing unit 120. Pacing signal generator 124 is configured to deliver electrical stimulation signals to ventricle 21 via electrodes 111, 112. Processing unit 120 may control pacing signal generator 124 to generate and deliver electrical stimulation to ventricle 21 via electrodes 111, 112. Electrical stimulation may include pacing pulses. Processing unit 120 may control pacing signal generator 124 to deliver electrical stimulation therapy using the pacing information described above and below, according to one or more therapy programs including pacing parameters, which may be stored in data memory 122. The pacing information is produced by the processing unit 120 based on the determined ventricular pacing time value and/or the ventricular pacing rate value, wherein the ventricular pacing time value and/or the ventricular pacing rate value is calculated by the processing unit according to the actual state adopted by the processing unit (see below).

Detector 126 may further include circuits that acquire time-dependent electrical signals (e.g. electric depolarization and repolarization signals) from the heart including intrinsic cardiac electrical activity, such as intrinsic atrial events and, if applicable, intrinsic ventricular events. Detector 126 may filter, amplify, and digitize the acquired electrical events of the heart chambers contractions. Processing unit 120 may receive the detected intrinsic atrial events and, if applicable, the intrinsic ventricular events provided by detector 126.

Processing unit 120 may assess cardiac activity signals comprising the intrinsic atrial events and, if applicable, the intrinsic ventricular events received from the detector 126 and is configured to determine the (time-dependent) estimated cardiac rate.

ILP 10 may include a housing, anchoring fixation features, and the electrodes 111, 112. The housing may have a pill-shaped cylindrical form factor in some examples. The anchoring fixation features are configured to connect ILP 10 to heart 20. Anchoring fixation features may be fabricated from a shape memory material, such as Nitinol. In some examples, anchoring fixation features may connect ILP 10 to heart 20 within one of the chambers of heart 20. For example, as illustrated and described herein with respect to Fig. 1, anchoring fixation features may be configured to anchor ILP 10 to heart 20 within right ventricle 21. Although ILP 10 includes a plurality of anchoring fixation features/elements that are configured to stably engage ILP 10 to cardiac tissue in the right ventricle, it is contemplated that a pacemaker according to the present disclosure may be engaged with cardiac tissue in other chambers of a patient's heart 20 using other types of anchoring fixation features.

ILP 10 may include two electrodes 111, 112, although more than two electrodes may be included on a pacemaker in other examples. Electrodes 111, 112 may be spaced apart a sufficient distance to be able to detect various electrical signals generated by the heart 20, such as P-waves generated by atria and QRS complex generated by ventricles. The housing houses electronic components of ILP 10. Electronic components may include any discrete and/or integrated electronic circuit components that implement analog and/or digital circuits capable of producing the functions attributed to ILP 10 described above and below.

The communication unit 128 may enable ILP 10 to communicate with other electronic devices, such as a programmer or other external patient monitor. In some examples, the housing may house a coil and/or an antenna for wireless communication. Housing may also include the power source 132.

The processing unit 120 may be adapted to control pacing of the right ventricle 21 in the first state using the known VDD mode based on the intrinsic atrial signal containing atrial contractions and, if applicable, the intrinsic ventricular signal containing ventricular contractions. The counter of the processing unit 120 used to time the AV delay (for providing the ventricular pace signal) may also be used to measure intrinsic AV delays. The VDD pacing mode may be R-Sync in the ILP 10. This means that every cycle is synchronized by every used ventricular event (intrinsic ventricular contraction or ventricular pacing). It is also an atrial tracking mode. This means that every sensed atrial contraction can shift the timing. In other words, VDD is effectively both R-Sync and P-Sync. The timing of the next potential ventricular pacing signal is scheduled based on the most recent ventricular event and a targeted pacing interval (determined from a target cardiac rate). Sensed atrial contractions "re-schedule" the next pacing signal by starting an AV interval. The estimated cardiac rate is determined from the intrinsic atrial event and, if applicable, from the intrinsic ventricular event.

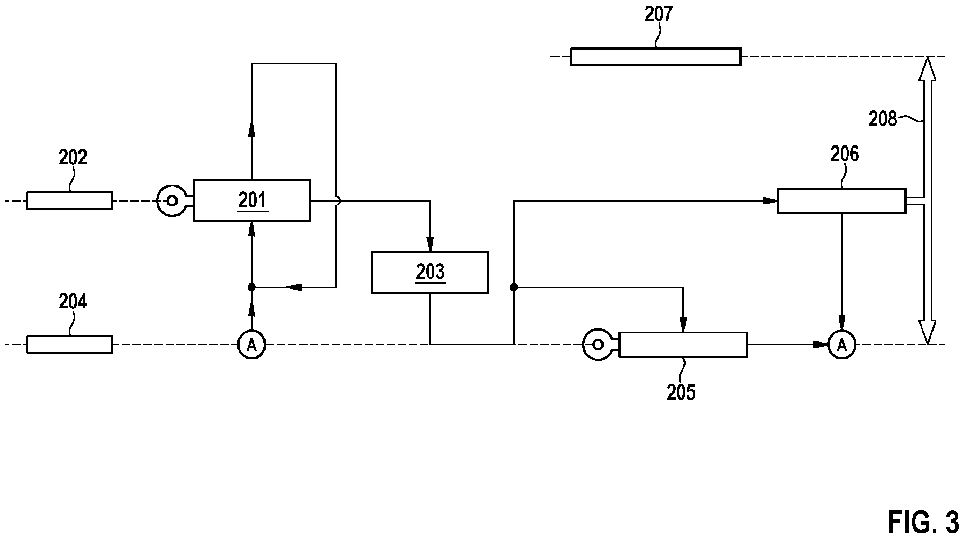

The method shown in diagram of Fig. 3 comprises controlling the pacing interval source, the tracking interval behavior, the motion circuit (enabled/disabled), the atrial tracking behavior (atrial sensing enabled/disabled), and rate decay behaviors under the various conditions that may arise while the pacemaker is programmed to the VDD mode. In the following, the embodiment refers to the determination of a ventricular pacing rate value by the processing unit but may function with regard to the determination of the ventricular pacing time value in an analogous way. The ideal behavior in the VDD mode is to have the ventricular event (either an intrinsic ventricular contraction or a ventricular pacing signal if the intrinsic AV conduction is inadequate) track the intrinsic atrial contractions (intrinsic atrial events). The ventricular pacing rate value is determined based on the estimated cardiac rate. In one embodiment, the ventricular pacing rate value is equal to the estimated cardiac rate, wherein the AV delay is calculated based on the estimated cardiac rate allowing the intrinsic ventricular contraction to occur prior to administering a pace output. If an intrinsic ventricular event occurs before the AV delay timeout, the pacemaker stops the AV delay and waits for the next atrial contraction in the atrial intrinsic signal. If the AV delay timeout occurs, a ventricular pacing signal is delivered. The (unchecked and checked) atrial tracking is symbolized by the 201 in Fig. 3. When the intrinsic atrial rate guides the VDD mode the estimated cardiac rate drives the operation of the pacemaker in an atrial tracking state 201. Subject to conditions where the estimated cardiac rate is greater than or transitions across a validity check rate (i.e., 202 in figure 3 which, for example, may represent a rate of 85 bpm) the system performs an assessment to determine whether or the apparent increased cardiac demand stems from patient body motion or is the result of a possible SVT. While tracking is desired in cases of patient motion, it is undesired and inappropriate subject to the presence of an active SVT. which may be, for example, As long as the estimated cardiac rate is less than the validity check rate (again, the rate designated by 202 in Figure 3), VDD mode pacing in the first state is continued (i.e. state 201). If the estimated cardiac rate is greater than or equal to the validity check rate, the system via triggering or periodicities checks whether the motion flag is on or off. The status of the motion flag is determined by the processing unit 120 using the motion signal as indicated above. The motion sensor provides an alternate reference for determining the source of increased cardiac demand (i.e., either from patient body motion or from an SVT).

If the check at validity check rate 201 determines that the increasing atrial rate might be an SVT, as indicated by discovering that the motion flag is false even though the cardiac rate is greater than or equal to the validity check rate, the processing unit is directed to transition to non-tracking states to avoid tracking the arrhythmia. The first change is that the ventricular pacing rate value and thereby the pacing is ramped down from the present estimated cardiac rate to the pre-defined first pacing rate threshold value (resting rate, see label 204) by switching to the SVT state (see step 203 in Fig. 3). If the resting rate (label 204) is reached, the processing unit continues with the non-tracking state (step 205) which provides VVI behavior, e.g. the ventricular pacing rate value remains at the resting rate. In the steps 203 and 205 atrial detections are disabled, and the motion sensor of the detector 126 is also disabled. After a predefined or user-selectable hold-off time period is expired the processing unit enters the FindSync state (step A in Fig. 3). The hold-off time period begins or initiates at either the Tracking Validity Check failure or once the resting rate is reached. The duration of this hold-off time period may be programmable and/or pre-defined and may last between 10 and 20 minutes, (e.g., 15 minutes). From the FindSync state the processing unit may transition back to the first atrial tracking state (again, labeled 201 in Figure 3) if synchronization with the intrinsic atrial events proves successful.

If the HCP has selected the option where the motion sensor can be used as a secondary rate response source, the non-tracking with motion state (206) is entered after the rate of the SVT state (203) first declines to the resting rate (as designated by 204 within Figure 3 This behavior ultimately renders a VVI-R mode therapy wherein the non-tracking with motion state serves as an alternative of the non-tracking state (i.e., state 205 which, by contrast renders a VVI mode therapy). In the non-tracking with motion state, the atrial sensing system is disabled, and the motion sensor is enabled. Since neither of the non-tracking states (i.e., 205 and 206) provides atrial tracking, the upper tracking rate mechanism is irrelevant. When the motion sensor is being used (i.e., as in state 206), the maximum pacing rate is the programmed maximum sensor rate (see label 207 in Figure 3). The double arrow (208) in Fig. 3 indicates that the ventricular pacing rate value when operating in state 206 (i.e., a VVI-R behavior) may, subject to driving inputs from the motion sensor, in turn, vary between the resting rate (label 204) and the maximum sensor rate (label 207). The specific rate adopted by the pacer within this range is directly dependent upon the motion level of the patient as detected by the motion sensor (e.g., an accelerometer) of the detector 126.

The next state for either of the non-tracking states (again, 205 and 206) is the Find Sync state (state A in Fig. 3). If the non-tracking with motion state (206) is allowed by the processing unit 120, the processing unit denies transitions to FindSync (again, state A) until the sensor rate is at or below the resting rate. After a predefined hold-off time period expires, the processing unit continues with the FindSync state (step A in Fig. 3) as explained above. In the FindSync state the processing unit synchronizes the pacing signals provided by the pacing signal generator by initiating AV delays subject to the detection of intrinsic atrial events. Accordingly, the processing unit attempts to determine the estimated cardiac rate which forms the basis for the ventricular pacing rate. From the FindSync state the processing unit 120 may continue with the first state (step 201) as indicated above subject to a robust detection of a reliable estimated cardiac rate.

The method shown in diagram of Fig. 3 comprises 1.) Tracking validity checks that occurs on transitions from a non-validated tracking states (first state in which the estimated cardiac rate is less than the validity check rate) to a validated tracking state (first state in which the estimated cardiac rate is or was previously equal to or greater than the validity check rate and the motion flag is true) and also optionally as periodic checks in validated tracking states (as discussed in 5. Below), 2.) Passing the tracking validity check (i.e., the estimated cardiac rate is or was previously equal to or greater than the validity check rate and the motion flag is true) allows entry into a validated tracking state while failure (motion flag is false) forces the implant to ramp down its ventricular pacing rate value to the resting rate, 3.) After hitting the resting rate, the HCP's configuration on the "use" or "non-use" of motion signaling (e.g., potentially implemented using a non-tracking mode parameter which may have a user-selectable first value or second value) determines whether VVI-R or VVI behavior will emerge, 4.) after a pre-defined hold-off time the state having VVI-R behavior or the state having VVI behavior is exited and the method is continued at "A" which begins the process of trying to reestablish a tracking condition (FindSync state), and 5.) periodically the first state having the validated tracking condition invalidates itself to allow for the pacemaker to reengage with the tracking validity check (i.e., to repeat the comparison of the estimated cardiac rate with the validity check rate and, if the estimated cardiac rate is equal to and greater than the estimated cardiac rate, the status of the motion flag is checked).

## Claims

1. A cardiac pacemaker (10) comprising a processing unit (120), a detector (126) and a pacing signal generator (124), wherein the detector (126) and the pacing signal generator (124) are electrically connected to the processing unit (120), wherein the processing unit (120) is configured to determine a ventricular pacing time value and/or a ventricular pacing rate value and to transmit a corresponding pacing information to the pacing signal generator (124) for providing a pacing signal for a patient's heart (20) based on this information, and comprises a motion flag reflecting the actual activity of the patient (30), an actual status of which is either true during high activity or false during lower activity and/or zero activity of the patient, wherein the detector (126) is configured to capture time-dependent signals of the cardiac activity containing intrinsic atrial events or evidence of such events from conducted intrinsic ventricular events of the patient's heart,
wherein in a first atrial tracking state (201) the processing unit (120) is configured to continuously determine an estimated cardiac rate based on at least one actual intrinsic atrial event or evidence of such an event from a conducted intrinsic ventricular event detected from the received cardiac activity signals and to compare the estimated cardiac rate with a pre-defined validity check rate (202), wherein if the estimated cardiac rate is greater than or equal to the validity check rate (202) the processing unit (120) is configured to assess the actual status of the motion flag,
- wherein, if the estimated cardiac rate is greater than or equal to the validity check rate (202) and the actual status of the motion flag is true or if the estimated cardiac rate is lower than the validity check rate (202), the processing unit (120) is configured to stay in the first atrial tracking state (201) in which the ventricular pacing time value and/or the ventricular pacing rate value is determined based on the estimated cardiac rate,
- wherein, if the estimated cardiac rate is greater than or equal to the validity check rate (202) and the actual status of the motion flag is false, the processing unit (120) is configured to transition into a supraventricular tachycardia state (SVT state, 203) in which the processing unit (120) is configured to ramp the ventricular pacing time value up and/or the ventricular pacing rate value down until a predefined first pacing time threshold value and a pre-defined first pacing rate threshold value (204), respectively, is reached, and to suspend detecting intrinsic atrial events of the patient's heart from the received cardiac activity signals.

2. The pacemaker of claim 1, wherein in the SVT state (203) is configured to suspend the capture of the time-dependent signals of the atrial cardiac activity.

3. The pacemaker of any one of the previous claims, wherein in the SVT state (203) after the ventricular pacing time value and/or the ventricular pacing rate value has reached the first pacing time threshold value and the first pacing rate threshold value (204), respectively, the processing unit (120) is configured to transition into a non-tracking state (205, 206), wherein in the non-tracking state (205, 206) the processing unit (120) is configured to continue suspending the detection of intrinsic atrial events of the patient's heart (20) from the received cardiac activity signals and/or to continue suspending the capture of the time-dependent signals of the atrial cardiac activity by the detector (126).

4. The pacemaker of claim 3, wherein in the non-tracking state (205, 206) the processing unit (120) is configured such that the ventricular pacing time value and/or the ventricular pacing rate value is determined using a VVI behavior (205), if a non-tracking-mode parameter has a pre-defined first value, or using a VVI-R behavior (206), if the non-tracking-mode parameter has a pre-defined second value different from the first value.

5. The pacemaker of claim 4, wherein the processing unit (120) is configured such that the ventricular pacing time value and/or the ventricular pacing rate value is determined using the VVI-R behavior (206) based on the recently detected and received motion signal provided by the detector (126) and/or based on the actual status of the motion flag and such that the ventricular pacing time value and/or the ventricular pacing rate value is determined using the VVI behavior (205) based on a pre-defined second pacing time threshold value and a pre-defined second pacing rate threshold value, respectively.

6. The pacemaker of any one of the claims 3 to 5, wherein in the non-tracking state (205, 206) after the ventricular pacing time value and/or the ventricular pacing rate value has not exceeded or undercut the first or second pacing time threshold value and the first or second pacing rate threshold value, respectively, for a pre-defined hold-off time period, the processing unit (120) is configured to transition the pacemaker into a state that attempts to reestablish atrial synchrony, wherein the detection of intrinsic atrial events of the patient's heart (20) from the received cardiac activity signals and/or the capture of the time-dependent signals of the atrial cardiac activity is resumed.

7. The pacemaker of claim 6, wherein the detector (126) is further configured to capture time-dependent signals of the cardiac activity containing intrinsic ventricular events, wherein the processing unit (120) is configured such that it synchronizes the pacing signals provided by the pacing signal generator (124) by means of AV delays in response to detected intrinsic atrial events in a transition state (state A) which is adopted after leaving the non-tracking state (205, 206) and prior entering the first atrial tracking state (201), wherein in the transition state (again, state A) the processing unit (120) is configured to resume the detection of intrinsic atrial events of the patient's heart (20) from the received cardiac activity signals and/or the capture of the time-dependent signals of the atrial cardiac activity.

8. An operation method of a cardiac pacemaker (10), wherein the pacemaker comprises a processing unit (120), a detector (126) and a pacing signal generator (124), wherein the detector (126) and the pacing signal generator (124) are electrically connected to the processing unit (120), wherein the processing unit (120) determines a ventricular pacing time value and/or a ventricular pacing rate value and transmits a corresponding pacing information to the pacing signal generator (124) for providing a pacing signal for a patient's heart (20) based on this information and comprises a motion flag reflecting the actual activity of the patient (30), an actual status of which is either true during high activity or false during lower activity and/or zero activity of the patient, wherein time-dependent signals of the cardiac activity containing intrinsic atrial events or evidence of such events from conducted intrinsic ventricular events of the patient's heart are captured by the detector (126),
wherein in a first atrial tracking state (201) continuously an estimated cardiac rate is determined based on at least one actual intrinsic atrial event or evidence of such event from at least one conducted intrinsic ventricular event detected from the received cardiac activity signals and the estimated cardiac rate is compared with a pre-defined validity check rate (202), wherein if the estimated cardiac rate is greater than or equal to the validity check rate (202) the actual status of the motion flag is assessed,
- wherein, if the estimated cardiac rate is greater than or equal to the validity check rate (202) and the actual status of the motion flag is true or if the estimated cardiac rate is lower than the validity check rate (202), the method continues in the first atrial tracking state (201) in which the ventricular pacing time value and/or the ventricular pacing rate value is determined based on the estimated cardiac rate,
- wherein, if the estimated cardiac rate is greater than or equal to the validity check rate (202) and the actual status of the motion flag is false, the method transitions into an SVT state (203) in which the ventricular pacing time value is ramped up and/or the ventricular pacing rate value is ramped down until a pre-defined first pacing time threshold value and a pre-defined first pacing rate threshold value(204), respectively, is reached, and the detection of intrinsic atrial events of the patient's heart from the received cardiac activity signals is suspended.

9. The method of claim 8, wherein in the SVT state (203) suspends the capture of the time-dependent signals of the atrial cardiac activity.

10. The method of any one of the claims 8 to 9, wherein in the SVT state (203) after the ventricular pacing time value and/or the ventricular pacing rate value has reached the first pacing time threshold value and the first pacing rate threshold value (204), respectively, the processing unit (120) transitions into a non-tracking state (205, 206), wherein in the non-tracking state (205, 206) the processing unit (120) continues suspending the detection of intrinsic atrial events of the patient's heart (20) from the received cardiac activity signals and/or continues suspending the capture of the time-dependent signals of the atrial cardiac activity by the detector (126).

11. The method of claim 10, wherein in the non-tracking state (205, 206) the ventricular pacing time value and/or the ventricular pacing rate value is determined using a VVI behavior (205), if a non-tracking-mode parameter has a pre-defined first value, or using a VVI-R behavior (206), if the non-tracking-mode parameter has a pre-defined second value different from the first value.

12. The method of claim 11, wherein the ventricular pacing time value and/or the ventricular pacing rate value is determined using the VVI-R behavior (206) based on the recently detected and received motion signal provided by the detector (126) and/or based on the actual status of the motion flag and/or the ventricular pacing time value and/or ventricular pacing rate value is determined using the VVI behavior (205) based on a pre-defined second pacing time threshold value and a pre-defined second pacing rate threshold value, respectively.

13. The method of any of claims 10 to 12, wherein in the non-tracking state (205, 206) after the ventricular pacing time value and/or the ventricular pacing rate value has not exceeded or undercut the first or second pacing time threshold value and the first or second pacing rate threshold value, respectively, for a pre-defined hold-off time period, the processing unit (120) dcauses the pacemaker to transition to a state that attempts to reestablish atrial synchrony, wherein the detection of intrinsic atrial events of the patient's heart (20) from the received cardiac activity signals and/or the capture of the time-dependent signals of the atrial cardiac activity is resumed.

14. A computer program product comprising instructions which, when executed by a processing unit (120), cause the processing unit (120) to perform the steps of the method according to any of the claims 8 to 13.

15. Computer readable data carrier storing a computer program product according to claim 14.
